# FASCICULE DE BREVET EUROPEEN

(11) **EP 0 658 596 B1**
(45) Date de publication et mention de la délivrance du brevet: **06.06.2001**
(21) Numéro de dépôt: 94402835.6
(22) Date de dépôt: 09.12.1994
(51) Int. Cl.: C08L 5/00, C09K 7/02, A61K 47/36, A23L 1/05

(54) **Composition à base de biopolymères à hydratation rapide**
Zusammensetzung auf Basis von Biopolymeren mit schneller Hydratation
Composition based on biopolymers with rapid hydration

(30) Priorité: 14.12.1993 US 168264
(43) Date de publication de la demande: 21.06.1995
(73) Titulaire: RHODIA INC., Cranbury, New Jersey 08512 (US)
(72) Inventeur: Wong, Philip, Kendall Park, New Jersey 08824 (US)
(74) Mandataire: Fabre, Madeleine-France

(56) Documents cités:
- EP-A- 0 509 924
- FR-A- 2 600 267
- GB-A- 2 172 008
- US-A- 4 041 234
- DATABASE WPI Week 8844 Derwent Publications Ltd., London, GB; AN 88-312246 & JP-A-63 230 703 (TAIYO KAGAKU KK) , 27 Septembre 1988

## Description

La présente invention a pour objet une composition à base de biopolymères, notamment du type gomme xanthane ou succinoglycane, sans poussière et à hydratation rapide, et leur utilisation comme agents épaississants et stabilisants pour la préparation de formulations industrielles (industries du bâtiment, de la peinture, du papier, du textile, des phytosanitaires, du traitement des eaux, industrie pétrolière ...), alimentaires, cosmétiques (dentifrices), pharmaceutiques.

Les polysaccharides de haut poids moléculaire d'origine microbienne, ou biopolymères, obtenus par fermentation d'un hydrate de carbone assimilable par un microorganisme approprié sont bien connus. L'exemple le plus représentatif en est la gomme xanthane. En raison de leurs propriétés épaississantes et de leur rhéologie, ils ont trouvé des applications variées, notamment dans le domaine alimentaire (tel que les produits laitiers, les boissons et les aliments à haute teneur en protéines) ou pharmaceutique, et dans les industries du bâtiment, de la peinture, du papier, du textile, des cosmétiques, des phytosanitaires, dans les produits de nettoyage et d'entretien industriels et ménagers, dans le traitement des eaux et dans l'industrie pétrolière, par exemple pour le forage et la récupération assistée du pétrole.

Pour de nombreuses applications, il est nécessaire de mettre le biopolymère sous forme d'une solution aqueuse faiblement concentrée ; il est connu que l'inconvénient majeur des poudres de biopolymères est leur difficulté à se dissoudre rapidement même sous agitation à effet de cisaillement élevé. En outre ces poudres sous leur forme commercialle actuelle, posent des problèmes de sécurité en raison des particules fines responsables de nuages de poussière.

C'est la raison pour laquelle il serait avantageux de développer une composition à base de biopolymère, notamment de type gomme xanthane ou succinoglycane, réellement sèche et sans poussière, pouvant être hydratée facilement lorsqu'elle est prête à l'emploi.

Une composition à base de gomme xanthane à hydratation rapide serait avantageuse si l'on pouvait la mélanger plus facilement à la formulation alimentaire, pharmaceutique ou industrielle finale. A l'heure actuelle, la gomme xanthane est disponible sous une forme nécessitant une agglomération. Cette façon de procéder est coûteuse et exige des investissements importants. En outre les compositions obtenues par granulation conduisent à une chute de densité du produit, ce qui entraîne des problèmes de stockage (volumes de stockage plus importants) pour les utilisateurs. La composition à base de biopolymère selon la présente invention permet d'abaisser les coûts de fabrication en évitant lesdits procédés d'agglomération qui sont coûteux.

Par ailleurs, un produit sans poussière est avantageux du point de vue santé ; il réduit, en effet, les particules en suspension dans l'air qui peuvent être respirées par les ouvriers dans l'industrie, diminue le risque d'explosion durant le stockage, évite les pertes de produit durant le stockage et le transport. Un produit sans poussière réduira aussi le nombre d'accidents dus aux glissades sur du produit répandu par terre.

Historiquement, lorsque l'on veut obtenir un obtenir une poudre sans poussière à hydratation rapide, le procédé mis en oeuvre pour y parvenir est l'agglomération. Ce procédé, toutefois, est coûteux et augmente, par conséquent, le coût du produit final. Le brevet US-A-4,041,234 de Maske décrit des complexes glyoxal-gomme xanthane dispersables, dans lesquels le glyoxal est ajouté au moût de fermentation de la gomme xanthane pour en améliorer la dispersabilité. C'est un procédé qui non seulement est coûteux, mais qui peut aussi poser des problèmes de relargage dans l'environnement du glyoxal après hydrolyse des ponts hémiacétals.

Le brevet US-A-4,299,825 de Lee décrit des solutions de gomme xanthane concentrées de haute viscosité mettant en oeuvre de nombreuses étapes d'ultrafiltration. Toutefois, elles ne contiennent que de 8 à 15 % de gomme xanthane existant sous forme de solution et non pas de particules hydratables sans poussière. Le brevet US-A-4,670,550 de Bleeker et al. décrit des émulsions de biopolymères analogues aux compositions de gomme xanthane faisant appel à des techniques de cisaillement important alors que le brevet US-A-4,254,257 de Schroeck décrit la précipitation des sels d'amine de gomme xanthane à partir du moût de fermentation.

Le brevet US-A-4,269,974 de Wintersdorff décrit une composition à base de gomme xanthane coulant facilement, composition dans laquelle la gomme est homogénéisée avec de l'huile végétale et de l'eau. Tous ces documents reconnaissent, cependant, la nécessité de cisailler la gomme pour l'hydrater.

Le brevet US-A-4,248,262 de Warren décrit un agent qui permet d'augmenter la viscosité à retardement sans formation de grumeaux et qui contient de la gomme xanthane encapsulée dans un revêtement constitué d'un corps gras choisi dans le groupe comprenant les acides gras, les mono et diglycérides d'acides gras et un tensio-actif choisi dans le groupe comprenant les sels alcalins des acides gras, des esters de sorbitan et d'acides gras, les alcools éthoxylés linéaires, les esters de saccharose et d'acides gras et leurs mélanges. Le tensio-actif sert à augmenter la solubilité du revêtement et à favoriser le mouillage de l'acide gras sur la particule. Le revêtement lui-même a une balance hydrophile/lipophile comprise entre 3,0 et 10,0.

Le brevet US-A-4,654,086 de Baird et al. décrit et revendique une composition à base de gomme xanthane sèche qui serait dispersable et hydratable, contenant de la gomme xanthane mélangée à un tensio-actif, tels que des monoglycérides acétylés, des esters de glycérol, des esters de sorbitan et autres similaires. Eventuellement, on y ajoute du sucre, mais le tensio-actif doit être ajouté en une quantité d'au moins 5 %, cette quantité pouvant aller jusqu'à 20 %. Toutefois, il se pose un problème, à savoir que, dans les applications alimentaires notamment, le tensio-actif peut posséder à ces concentrations un goût indésirable qui peut le rendre inutilisable dans la plupart des produits alimentaires. Si de telles compositions sont utilisées pour la préparation de formulations industrielles (agrochimiques, cosmétiques, peintures...), la présence d'une quantité élevée de tensio-actifs peut engendrer des problèmes de moussage, d'incompatibilité avec les autres tensio-actifs présents dans les formulations finales ou modifier les propriétés applicatives desdites formulations. Baird et al. reconnaissent, cependant, que, au-dessous de 5 %, les problèmes relatifs à l'hydratation et à la dispersabilité se posent de nouveau.

Un autre type de biopolymères présentant des propriétés épaississantes et de rhéologie adaptées aux mêmes types d'applications que celles de la gomme xanthane, est constitué par les succinoglycanes dont le motif de base contient du glucose, du galactose et un reste succinyle; ils sont décrits dans les demandes de brevet européen EP-A-351 303 et 40 445, ainsi que dans Carbohydrate Research, 73 (1979) pp.159-168, de Clarence A. Knutson ; ils peuvent être obtenus par fermentation microbienne d'un milieu comportant une source de carbone, au moyen d'un microorganisme appartenant au genre *Arthrobacter,* tel que *Arthrobacter stabilis,* en particulier la souche *Arthrobacter stabilis* NRRL-B-1973, au genre *Agrobacterium,* tels *Agrobacterium tumefaciens, Agrobacterium radiobacter* ou, *Agrobacterium rhizogenes,* au genre *Rhizobium,* en particulier *Rhizobium meliloti* et *Rhizobium trifoli*, au genre *Alcaligenes* tel *Alcaligenes faecalis,* en particulier la variété myxogenes ou au genre *Pseudomonas,* en particulier les souches *Pseudomonas sp*. NCIB 11264 et NCIB 11592. Parmi ces succinoglycanes, on peut tout particulièrement citer les gommes rhéozan, décrites dans la demande de brevet européen EP-A-351 303, et obtenues par fermentation d'une source carbonée au moyen de la souche *Agrobacterium tumefaciens* 1-736 déposée à la collection Nationale de culture des Microorganismes (CNCM).

La présente invention a pour objet de mettre à disposition une poudre d'hydrocolloïde de biopolymère stable, réellement sans poussière, entièrement hydratable et pouvant être utilisée comme épaississant et agent de suspension. La présente invention a pour autre objet de mettre à disposition une composition à base de biopolymère entièrement hydratable, réellement sans poussière, contenant un biopolymère et de très faibles quantités de tensio-actif, de façon à ce que ce dernier ne puisse pas être détecté dans les applications finales. La présente invention a encore pour objet la préparation d'une composition à base de biopolymère sans poussière, réellement stable, entièrement hydratable et efficace comme épaississant et agent de suspension dans les applications alimentaires, pharmaceutiques et industrielles.

Une composition à base de biopolymère (polysaccharide d'origine microbienne) hydratable, réellement sans poussière, assure d'excellentes propriétés épaississantes et d'agent de suspension lorsqu'elle est mise en oeuvre dans des applications alimentaires, pharmaceutiques et industrielles. Le biopolymère se trouve également stabilisé et ne requiert pas un degré important de cisaillement et/ou d'agitation pour l'amener à se disperser en solution. La composition est constituée d'un biopolymère et d'une faible quantité de tensio-actif choisi dans le groupe des tensio-actifs anioniques mouillants et des tensio-actifs non-ioniques à HLB élevé (au moins 7), tels que les esters de sorbitan éthoxylés, les esters de glycérol, les mono et diglycérides et autres similaires.

Alors que, dans l'état antérieur de la technique, on a déjà cherché à produire une poudre de gomme xanthane hydratable, il n'a jamais été possible d'y parvenir avec de faibles quantités d'additifs et sans avoir à recourir à des procédés d'agglomération coûteux. Or, on a découvert de façon surprenante et inattendue que, en mettant en oeuvre un biopolymère comme une gomme xanthane spécifique qui est le produit de fermentation du *Xanthomonas campestris* et qui est vendue sous la marque Rhodigel® Rhodopol® et Rhodicare® ou comme un succinoglycane qui est le produit de fermentation de la souche *Agrobacterium tumefaciens* et qui est vendu sous la marque Rhéozan® (commercialisés par Rhône-Poulenc) on peut obtenir en n'utilisant qu'une faible quantité de tensio-actif une composition à base de biopolymère qui possède un excellent pouvoir d'hydratation, épaississant, stabilisant et d'agent de suspension.

Sans aucun rapport avec une quelconque théorie et alors que les gommes xanthanes et les succinoglycanes de l'état antérieur de la technique ont des formes globulaires ou ovoïdes à surface lisse lorsqu'on les observe au microscope, la gomme xanthane et le succinoglycane mis en oeuvre dans les compositions de la présente invention possèdent des arêtes déchiquetées et rugueuses ainsi que des surfaces irrégulières. De plus, bien que toutes les gommes xanthanes soient caractérisées chimiquement comme étant des polyglucomannanes, celles qui sont utilisées dans la présente invention sont de préférence les sels de sodium de ces derniers. Il est possible que ces particularités de la gomme en augmentent la réactivité avec le tensio-actif et aboutissent aux propriétés surprenantes et inattendues de la composition permettant ainsi d'utiliser moins de tensio-actif pour atteindre les objectifs fixés.

Le tensio-actif utilisé de préférence est un tensio-actif anionique mouillant ou non-ionique de balance hydrophile/lipophile (HLB) élevée (au moins 10). Les valeurs de ce HLB peuvent être tout particulièrement comprises entre environ 10 et environ 16.

Parmi ces tensio-actifs on peut citer :
- les savons des acides gras tels que les sels de sodium ou de potassium d'acides gras saturés ou insaturés en C₆-C₂₄ ou de dérivés d'acides aminocarboxyliques comme le N-lauryl sarconisate de sodium,
- les sulfates d'alcoylphénols polyoxyéthylénés ; les sulfates d'arylalkylphénols polyoxyéthylénés,
- les esters phosphoriques de dérivés oxyéthylénés tels que les phosphates d'alcools gras polyoxyéthylénés ; les phosphates d'alcoylphénols polyoxyéthylénés, les phosphates d'arylalkylphénols polyoxyéthylénés,
- les alcoylsulfonates, par exemple les alkylsulfoesters des acides en C₄-C₃₀ du type diakylsulfosuccinate de sodium ; les alcoylbenzènesulfonates comme le nonylbenzènesulfonate de sodium et le dodecylbenzènesulfonate de sodium ; les lignosulfonates,
- les alcoylphénols ou alcoylarylphénols polyoxyéthylénés comme les nonylphénols polyoxyéthylénés et les dodécylphénols polyoxyéthylénés, les tristyrylphénols polyoxyéthylénés
- les alcools gras et acides gras polyoxyéthylénés et polyoxypropylénés,
- les alcanolamides d'acides gras polyoxyéthylénés et polyoxypropylénés,
- les esters de polyols, comme les esters de glycérol (tensio-actifs alimentaires) ou de propylène glycol, des acides gras, des huiles et graisses alimentaires, des mélanges d'acides gras et d'acide acétique et/ou lactique et/ou citrique et/ou tartrique ;
- les esters de saccharose tels que les sucro-esters et les sucroglycérides (tensio-actifs alimentaires) ; les esters d'acides gras de sorbitan (tensio-actifs alimentaires) ; et leurs dérivés polyoxyéthylénés et polyoxypropylénés comme les esters de polyéthyléneglycol ou de polypropylèneglycol polyoxyéthylénés, les esters de sorbitan polyoxyéthylénés, les esters d'acide tartrique polyoxyéthylénés, les glycérides oléiques polyoxyéthylénés,
- la lécithine (tensio-actif alimentaire).
De préférence ledit tensio-actif est choisi parmi les esters de sorbitan polyoxyéthylénés ou tout autre tensio-actif ci-dessus se présentant sous forme liquide dans les conditions de préparation de la composition de l'invention.

Le tensio-actif est combiné avec le biopolymère en quantités relativement faibles, c'est-à-dire comprises entre environ 0,1 % et environ 3,0 % par rapport au poids total de la composition. La teneur en tensio-actif est comprise de préférence entre environ 0,1 % et environ 1,5 % en poids par rapport au poids total de la composition à base de biopolymère.

Il est également surprenant et inattendu de constater avec quelle facilité le biopolymère et l'émulsifiant se mélangent entre eux. On dispose simplement la gomme xanthane ou le succinoglycane dans un mélangeur Hobart standard ou dans tout autre mélangeur similaire et on y ajoute l'émulsifiant. Cette opération est réalisée à une température de l'ordre de 15 à 40°C. On parachève le mélange à vitesse lente pendant environ cinq minutes à l'aide d'un mélangeur à palettes. Le produit final est homogène.

Les compositions biopolymères/tensio-actif selon la présente invention sont hautement visqueuses, possèdent une texture homogène et peuvent couler. Elles sont également stables au stockage. On peut encore améliorer leur coulabilité en y incorporant de la silice, des phosphates (hexametaphosphte de sodium ...) en quantité de l'ordre de 0,5% par exemple.

Ces caractéristiques permettent de les incorporer facilement dans de nombreuses formulations alimentaires, pharmaceutiques, cosmétiques (notamment dans les dentifrices) ou industrielles (dans les formulations pour le nettoyage de surfaces par exemple) et d'abaisser, par conséquent, les coûts de production tout en augmentant les vitesses de production.
Ces compositions sont également intéressantes pour la stabilisation de formulations agrochimiques, du type suspensions concentrées ("flowables") ; il est en effet possible d'additionner directement la quantité de composition biopolymère/tensio-actif nécessaire à la stabilisation de la formulation (généralement entre 0,2 et 0,3 % par rapport à la formulation finale) sans dilution intermédiaire de ladite composition.
Ces compositions étant stables thermiquement et stables en milieu basique (à pH de l'ordre de 9) ainsi qu'en milieu salin, sont particulièrement bien adaptées à la formulation de boue à l'eau destinée au forage.

Les exemples suivants sont donnés à titre illustratif.

### Exemple 1

### Compositions Rhodigel® /Polysorbate 60®

On prépare les quatre compositions à base de Rhodigel® (gomme xanthane alimentaire présentant une taille moyenne de particules de l'ordre de 180µm - 80 mesh - obtenue par action du *Xanthomonas campestris* ATCC 13951) et de Polysorbate 60® (monostéarate de sorbitan polyoxyéthylèné commercialisé par Rhône-Poulenc) figurant au tableau 1 en mettant en oeuvre le procédé de mélange standard indiqué plus haut ; dans ce tableau ne figurent que les pourcentages de Polysorbate 60® , indiqués par rapport au poids total de la composition gomme xanthane/tensio-actif.
On met chacune de ces compositions en solution à 1% en poids dans de l'eau distillée. Cette mise en solution est réalisée par mélange à l'aide d'un mélangeur à broches (spindle mixer) équipé d'une lame de 6,35 cm et tournant à 1000 tours/min pendant 1 minute.
Après arrêt de l'agitation, on mesure la viscosité en mPa.s. de ladite solution, à l'aide d'un viscosimètre de Brookfield, modèle LVT, broche n° 3, réglé à 12 tours/min.
A nouveau - on agite la solution pendant 1 minute, et on mesure la viscosité après l'arrêt de l'agitation,
- puis on agite la solution pendant 2 minutes, et on mesure la viscosité après l'arrêt de l'agitation,
- et enfin on agite la solution pendant 4 minutes, et on mesure la viscosité après l'arrêt de l'agitation.

Les valeurs des viscosités mesurées à ces quatre moments différents figurent au tableau 1 suivant.

**TABLEAU 1**

| **Viscosités (mPa.s.)** | | | | |
|---|---|---|---|---|
| **% Polysorbate 60**® | **1 minute** | **2 minutes** | **4 minutes** | **8 minutes** |
| 0,00 | 1 400 | 2 300 | 3 500 | 4 500 |
| | gros grumeaux | Gros grumeaux | Gros grumeaux | Gros grumeaux |
| 0,50 | 3 700 | 4 400 | 4 600 | 4 800 |
| | Petits grumeaux | Petits grumeaux | Très petits grumeaux | Pas de grumeaux |
| 0,75 | 3 900 | 4 500 | 4 800 | 4 800 |
| | Petits grumeaux | Petits grumeaux | Pas de grumeaux | Pas de grumeaux |
| 1,00 | 4 200 | 4 500 | 4 800 | 4 800 |
| | Petits grumeaux | Très petits grumeaux | Pas de grumeaux | Pas de grumeaux |

On constate :
- qu'au bout de 2 minutes, la viscosité de la solution dépasse 90% de sa viscosité finale pour un taux de Polysorbate 60® de 0,5 à 1%;
- que la vitesse d'hydratation (évolution de la viscosité en fonction du temps) a été améliorée sans modifier la viscosité finale du produit).

### Exemple 2

### Composition Rhodopol 23® /Igepal BC 10®

On prépare les 3 compositions Rhodopol 23® /Igepal BC 10® (nonylphénol contenant 10 motifs oxyéthylène - 10 OE - de HLB = 13,3 , commercialisé par Rhône-Poulenc), figurant au tableau 2, puis 3 solutions à 1% dans l'eau distillée, selon le mode opératoire de l'exemple 1.

La mise en solution est réalisée sur une quantité de 400ml dans un bécher de 800ml de forme basse, avec une pale cadre (longueur 700mm - largeur 35mm) avec une agitation de 500 tours/min.
On mesure comme à l'exemple 1 la viscosité en mPa.s. de ladite solution, à 4 moments différents, à l'aide d'un viscosimètre de Brookfield, modèle LVT, broche n° 3, réglé à 12 tours/min.
Les valeurs des viscosités mesurées à ces quatre moments différents figurent au tableau 2 suivant.

**TABLEAU 2**

| **Viscosités (mPa.s.)** | | | | |
|---|---|---|---|---|
| **% Igepal BC 10**® | **1 minute** | **2 minutes** | **4 minutes** | **8 minutes** |
| 0,00 | 1 900 | 2 700 | 4 600 | 4 800 |
| 0,50 | 3 200 | 3 900 | 4 300 | 4 500 |
| 1,00 | 3 000 | 3 700 | 4 200 | 4 300 |

### Exemple 3

### Composition Rheozan 23® /Igepal BC 10®

On répète les opérations décrites à l'exemple 2 en remplaçant le Rhodopol 23® par du Rheozan® .
Les valeurs des viscosités mesurées aux quatre moments différents figurent au tableau 3 suivant.

**TABLEAU 3**

| **Viscosités (mPa.s.)** | | | | |
|---|---|---|---|---|
| **% Igepal BC 10**® | **1 minute** | **2 minutes** | **4 minutes** | **8 minutes** |
| 0,00 | 1 000 | 3 600 | 6 700 | 7 900 |
| 0,50 | 2 700 | 4 900 | 7 600 | 8 000 |
| 1,00 | 2 700 | 5 300 | 7 600 | 8 000 |

### Exemple 4

### Composition Rhodopol 23® /Rhodasurf 860/P®

On répète les opérations décrites à l'exemple 2 en remplaçant l'Igepal BC 10® par le Rhodasurf 860/P® (alcool gras éthoxylé 6 OE homologué EPA - Environment Protection Agency - de HLB = 11, commercialisé par Rhône-Poulenc).
Les valeurs des viscosités mesurées aux quatre moments différents figurent au tableau 4 suivant.

**TABLEAU 4**

| **Viscosités (mPa.s.)** | | | | |
|---|---|---|---|---|
| **% Rhodasurf 860/P**® | **1 minute** | **2 minutes** | **4 minutes** | **8 minutes** |
| 0,00 | 1 900 | 2 700 | 4 600 | 4 800 |
| 0,50 | 3 900 | 4 200 | 4 500 | 4 700 |
| 1,00 | 3 600 | 4 200 | 4 400 | 4 500 |

### Exemple 5

### Composition Rheozan 23® /Rhodasurf 860/P®

On répète les opérations décrites à l'exemple 4 en remplaçant le Rhodopol 23® par du Rheozan® .
Les valeurs des viscosités mesurées aux quatre moments différents figurent au tableau 5 suivant.

**TABLEAU 5**

| **Viscosités (mPa.s.)** | | | | |
|---|---|---|---|---|
| **% Rhodasurf 860/P**® | **1 minute** | **2 minutes** | **4 minutes** | **8 minutes** |
| 0,00 | 1 000 | 3 600 | 6 700 | 7 900 |
| 1,00 | 3 200 | 4 800 | 7 300 | 7 600 |

### Exemple 6

### Composition Rhodopol 23® /Rhodasurf DB 311®

On répète les opérations décrites à l'exemple 2 en remplaçant l'lgepal BC 10® par le Rhodasurf DB 311® (alcool gras éthoxylé de HLB = 12, commercialisé par Rhône-Poulenc).
Les valeurs des viscosités mesurées aux quatre moments différents figurent au tableau 6 suivant.

**TABLEAU 6**

| **Viscosités (mPa.s.)** | | | | |
|---|---|---|---|---|
| **% Rhodasurf DB311**® | **1 minute** | **2 minutes** | **4 minutes** | **8 minutes** |
| 0,00 | 1 900 | 2 700 | 4 600 | 4 800 |
| 1,00 | 4 300 | 4 400 | 4 500 | 4 600 |

### Exemple 7

### Utilisation des compositions de l'exemple 2 dans les formulations pour forage pétrolier.

Pour juger de l'applicabilité des compositions de l'exemple 2 dans les formulations pour forage, on mesure la vitesse d'hydratation de ces compositions dans de l'eau de mer Atlantique.
Le matériel utilisé consiste en :
- un bécher de forme haute de 800ml
- un agitateur IKA muni d'une pale déflocculeuse de 65mm de diamètre tournant à 500 tours/min
- un appareil Coesfeld® de mesure de couple moteur de l'agitateur
- un enrégistreur graphique de la variation du couple moteur en fonction du temps.
Le mode opératoire est le suivant :
- on introduit 495g d'eau de mer Atlantique dans le bécher
- la pale déflocculeuse est centrée à 1cm du fond du bécher
- on met l'ensemble des appareils en mouvement
- on note la vitesse de déroulement du papier (echelle temps) de l'enrégistreur graphique
- on introduit la composition (5g) en 15 secondes sur le côté du vortex crée par l'agitation
- la mesure est arrêtée 20 minutes après l'addition de la composition.
L'enregistrement obtenu permet de définir l'évolution de la viscosité en fonction du temps.
Les valeurs mesurées sur l'enregistrement sont le décalage en mm entre le niveau d'enregistrement avant l'ajout de composition et celui à l'instant t après l'ajout (2,5min ; 5min; 10min ; 15min et 20min après l'ajout).
Ce décallage correspondant à une augmentation de viscosité est donné au tableau 7 suivant.

**Tableau 7**

| **mm de décalage à l'instant t** | | | | | |
|---|---|---|---|---|---|
| **% Igepal BC 10**® | **2,5min** | **5min** | **10min** | **15min** | **20min** |
| 0,00 | 4 | 11 | 24 | 40 | 54 |
| 0,50 | 16 | 28 | 47 | 58 | 65 |
| 1,00 | 16 | 29 | 48 | 59 | 66 |

## Revendications

1. Composition à base de gomme xanthane et d'un tensio-actif, caractérisée en ce que la gomme xanthane possède des arêtes déchiquetées et rugueuses, et des surfaces irrégulières, en ce que la quantité de tensioactif représente de l'ordre de 0,1 à 3 % en poids par rapport au poids total de la composition, et en ce que la composition est susceptible d'être obtenue en mettant en oeuvre les étapes suivantes :
- on dispose la gomme xanthane dans un mélangeur de type Hobart standard et on y ajoute le tensio-actif, à une température comprise entre 15 et 40°C.
- on parachève le mélange à vitesse lente en pendant environ 5 minutes à l'aide d'un mélangeur à palettes.

2. Composition selon la revendication 1, caractérisée en ce que la quantité de tensio-actif représente de l'ordre de 0,1 à 1,5 % en poids par rapport au poids total de la composition.

3. Composition selon l'une quelconque des revendications 1 ou 2, caractérisée en ce que la gomme xanthane est le produit de fermentation du *Xanthomonas campestris.*

4. Composition selon l'une quelconque des revendications précédentes, caractérisée en ce que la gomme xanthane le produit de fermentation du *Xanthomonas campestris* ATCC 13951.

5. Composition selon l'une quelconque des revendications précédentes caractérisée en ce que la gomme xanthane est un sel de sodium de polyglucomannane.

6. Composition selon l'une quelconque des revendications 1 à 5, caractérisée en ce que le tensio-actif est un tensio-actif mouillant anionique ou non ionique, de balance hydrophile/lipophile d'au moins 10.

7. Composition selon la revendication précédente, caractérisée en ce que la balance hydrophile/lipophile est comprises entre environ 10 et environ 16.

8. Composition selon l'une quelconque des revendications précédentes, caractérisée en ce que le tensio-actif est choisi parmi les savons des acides gras, les dérivés d'acides aminocarboxyliques, les sulfates d'alcoylphénols polyoxyéthylénés, les sulfates d'arylalkylphénols polyoxyéthylénés, les esters phosphoriques de dérivés oxyéthylénés, les phosphates d'alcoylphénols polyoxyéthylénés, les phosphates d'arylalkylphénols polyoxyéthylénés, les alcoylsulfonates, les alcoylbenzènesulfonates, les lignosulfonates, les alcoylphénols polyoxyéthylénés, les alcoylarylphénols polyoxyéthylénés, les alcools gras et les acides gras polyoxyéthylénés et polyoxypropylénés, les esters de polyols et leurs dérivés polyoxyéthylénés et polyoxypropylénés, les esters de saccharose, les esters d'acides gras de sorbitan et leurs dérivés polyoxyéthylénés et polyoxypropylénés, et la lécithine.

9. Composition selon l'une quelconque des revendications précédentes, caractérisée en ce que le tensio-actif est choisi parmi les esters de sorbitan polyoxyéthylénés, les nonylphénols polyoxyéthylénés, les alcools gras polyoxyéthylénés, les tristyrylphénols polyoxyéthylénés.

10. Composition selon l'une quelconque des revendications précédentes, caractérisée en ce que le tensio-actif est choisi parmi les esters de glycérol, les sucroesters, les sucroglycérides, les esters d'acide gras de sorbitan, la lécithine.

11. Composition selon l'une quelconque des revendications précédentes, caractérisée en ce que le tensio-actif est choisi parmi les tensioactifs liquides à une température de l'ordre de 15 à 40°C.

12. Utilisation de la composition telle que définie dans l'une quelconque des revendications 1 à 11, comme agent épaississant et stabilisant dans les formulations :
- agrochimiques,
- alimentaires ou pharmaceutiques,
- pour le forage et la récupération assistée du pétrole,
- pour nettoyage et entretien industriel et ménager.

13. Formulation agrochimique, alimentaire, pharmaceutique, ou pour nettoyage et entretien industriel et ménager, comprenant une composition définie selon l'une quelconque des revendications 1 à 11.

## Patentansprüche

1. Zusammensetzung auf Basis von Xanthangummi und einem oberflächenaktiven Mittel, dadurch gekennzeichnet, daß der Xanthangummi zerstückelte und rauhe Kanten und ungleichmäßige Oberflächen besitzt, und daß die Menge an oberflächenaktivem Mittel in der Größenordnung von 0,1 bis 3%, ausgedrückt als Gewicht, bezogen auf das Gesamtgewicht der Zusammensetzung, liegt, und daß die Zusammensetzung erhalten werden kann, indem man folgende Schritte durchführt:
- man legt den Xanthangummi in einem Mischer vom Standard-Hobart-Typ vor und gibt hierzu das oberflächenaktive Mittel bei einer Temperatur zwischen 15 und 40 °C;
- man vollendet den Mischvorgang bei langsamer Geschwindigkeit während etwa 5 Minuten mit Hilfe eines Schaufelmischers.

2. Zusammensetzung gemäß Anspruch 1, dadurch gekennzeichnet, daß die Menge des oberflächenaktiven Mittels in der Größenordnung von 0,1 bis 1,5%, ausgedrückt als Gewicht, bezogen auf das Gesamtgewicht der Zusammensetzung, liegt.

3. Zusammensetzung gemäß einem der Ansprüche 1 oder 2, dadurch gekennzeichnet, daß der Xanthangummi das Fermentationsprodukt von Xanthomonas campestris ist.

4. Zusammensetzung gemäß einem der vorhergehenden Ansprüche, dadurch gekennzeichnet, daß der Xanthangummi das Fermentationsprodukt von Xanthomonas campestris ATCC 13951 ist.

5. Zusammensetzung gemäß einem der vorhergehenden Ansprüche, dadurch gekennzeichnet, daß der Xanthangummi ein Natriumsalz von Polyglucomannan ist.

6. Zusammensetzung gemäß einem der Ansprüche 1 bis 5, dadurch gekennzeichnet, daß das oberflächenaktive Mittel ein anionisches oder nicht-ionisches benetzendes oberflächenaktives Mittel mit einer hydrophil/lipophil-Balance von mindestens 10 ist.

7. Zusammensetzung gemäß dem vorhergehenden Anspruch, dadurch gekennzeichnet, daß die hydrophil/lipophil-Balance zwischen etwa 10 und etwa 16 liegt.

8. Zusammensetzung gemäß einem der vorhergehenden Ansprüche, dadurch gekennzeichnet, daß das oberflächenaktive Mittel ausgewählt ist unter den Seifen der Fettsäuren, den Aminocarbonsäurederivaten, den Sulfaten der polyoxyethylenierten Alkylphenole, den Sulfaten der polyoxyethylenierten Arylalkylphenole, den Phosphorsäureestem der oxyethylenierten Derivate, den Phosphaten der polyoxyethylenierten Alkylphenole, den Phosphaten der polyoxyethylenierten Arylalkylphenole, den Alkylsulfonaten, den Alkylbenzolsulfonaten, den Lignosulfonaten, den polyoxyethylenierten Alkylphenolen, den polyoxyethylenierten Alkylarylphenolen, den polyoxyethylenierten und polyoxypropylenierten Fettalkoholen und Fettsäuren, den Estern der Polyole und deren polyoxyethylenierten und polyoxypropylenierten Derivaten, den Estern der Saccharose, den Fettsäuresorbitanestern und deren polyoxyethylenierten und polyoxypropylenierten Derivaten und dem Lecithin.

9. Zusammensetzung gemäß einem der vorhergehenden Ansprüche, dadurch gekennzeichnet, daß das oberflächenaktive Mittel ausgewählt ist unter den polyoxyethylenierten Sorbitanestern, den polyoxyethylenierten Nonylphenolen, den polyoxyethylenierten Fettalkoholen, den polyoxyethylenierten Tristyrylphenolen.

10. Zusammensetzung gemäß einem der vorhergehenden Ansprüche, dadurch gekennzeichnet, daß das oberflächenaktive Mittel ausgewählt ist unter den Estern des Glycerins, den Sucroestern, den Sucroglyceriden, den Sorbitanfettsäureestem, dem Lecithin.

11. Zusammensetzung gemäß einem der vorhergehenden Ansprüche, dadurch gekennzeichnet, daß das oberflächenaktive Mittel ausgewählt ist unter den bei einer Temperatur in der Größenordnung von 15 bis 40°C flüssigen oberflächenaktiven Mitteln.

12. Verwendung der Zusammensetzung gemäß einem der Ansprüche 1 bis 11 als Verdickungs- und Stabilisierungsmittel in den Formulierungen:
- agrochemischen,
- Nahrungsmittel- oder pharmazeutischen Formulierungen,
- für die unterstützte Bohrung und Förderung von Erdöl.
- für die industrielle Reinigung und Wartung und den Haushalt.

13. Agrochemische Nahrungsmittel- und pharmazeutische Formulierung oder Formulierung für die industrielle Reinigung und Wartung und den Haushalt, umfassend eine Zusammensetzung gemäß einem der Ansprüche 1 bis 11.

## Claims

1. A composition based on xanthan gum and a surfactant, characterized in that the xanthan gum has jagged and rough edges, and irregular surfaces, in that the quantity of surfactant represents of the order of 0.1% to 3% by weight with respect to the total composition weight, and in that the composition can be obtained by carrying out the following steps:
• placing the xanthan gum in a standard Hobart type mixer and adding a surfactant at a temperature in the range 15°C to 40°C;
• completing mixing at low speed for about 5 minutes using a paddle mixer.

2. A composition according to claim 1, characterized in that the quantity of surfactant represents of the order of 0.1% to 1.5% by weight with respect to the total composition weight.

3. A composition according to claim 1 or claim 2, characterized in that the xanthan gum is the fermentation product of *Xanthomonas campestris.*

4. A composition according to any one of the preceding claims, characterized in that the xanthan gum is the fermentation product of *Xanthomonas campestris* ATCC 13951.

5. A composition according to any one of the preceding claims, characterized in that the xanthan gum is a polyglucomannane sodium salt.

6. A composition according to any one of claims 1 to 5, characterized in that the surfactant is an anionic or non-ionic wetting surfactant with a hydrophilic/lipophilic balance of at least 10.

7. A composition according to the preceding claim, characterized in that the hydrophilic/lipophilic balance is in the range about 10 to about 16.

8. A composition according to any one of the preceding claims, characterized in that the surfactant is selected from fatty acid soaps, aminocarboxylic acid derivatives, polyoxyethylenated alkylphenol sulphates, polyoxyethylenated arylalkylphenol sulphates, phosphoric esters of oxyethylenated derivatives, polyoxyethylenated alkylphenol phosphates, polyoxyethylenated arylalkylphenol phosphates, alkylsulphonates, alkylbenzene sulphonates, lignosulphonates, polyoxyethylenated alkylphenols, polyoxyethylenated alkylarylphenols, fatty alcohols and polyoxyethylenated and polyoxypropylenated fatty acids, polyol esters and their polyoxyethylenated and polyoxypropylenated derivatives, saccharose esters, fatty acid esters of sorbitan and their polyoxyethylenated and polyoxypropylenated derivatives, and lecithin.

9. A composition according to any one of the preceding claims, characterized in that the surfactant is selected from polyoxyethylenated sorbitan esters, polyoxyethylenated nonylphenols, polyoxyethylenated fatty alcohols, and polyoxyethylenated tristyrylphenols.

10. A composition according to any one of the preceding claims, characterized in that the surfactant is selected from glycerol esters, sucroesters, sucroglycerides, fatty acid esters of sorbitan, and lecithin.

11. A composition according to any one of the preceding claims, characterized in that the surfactant is selected from surfactants that are liquid at a temperature of the order of 15°C to 40°C.

12. Use of a composition as defined in any one of claims 1 to 11 as a thickening and stabilising agent for the following formulations:
• agrochemicals;
• foodstuffs or pharmaceuticals;
• for drilling and assisted recovery of oil;
• for industrial and domestic cleaning and maintenance.

13. A formulation for agrochemical, food or pharmaceutical use or for industrial or domestic cleaning and maintenance, comprising a composition as defined in any one of claims 1 to 11.
